# DEMANDE DE BREVET EUROPEEN

(11) **EP 4 245 288 A1**
(43) Date de publication de la demande: **20.09.2023**
(21) Numéro de dépôt: 23161071.8
(22) Date de dépôt: 09.03.2023
(51) Int. Cl.: A61K 8/06, A61K 8/44, A61Q 19/00, A61K 8/92, C09K 23/28, C09K 23/38

(54) **UTILISATION COMME BASE ÉMULSIONNANTE DANS UNE FORMULATION COSMÉTIQUE OU PHARMACEUTIQUE, D'UNE COMPOSITION COMPRENANT DU STEAROYL GLUTAMATE**

(30) Priorité: 15.03.2022 FR 2202247
(71) Demandeur: GATTEFOSSE SAS, 69804 Saint-Priest Cedex (FR)
(72) Inventeur: NOLLET, Maxime, 69003 LYON (FR); BOULGHOBRA, Hakime, 69330 MEYZIEU (FR); RODIER, Jean-David, 69100 VILLEURBANNE (FR)
(74) Mandataire: Cabinet Laurent & Charras

(57) **Abrégé**

Utilisation comme base émulsionnante dans une formulation cosmétique ou pharmaceutique, d'une composition comprenant un mélange de stéaroyl glutamate de métal alcalin et d'acide stéaroyl glutamique, caractérisée en ce que le tensio-actif présente un pH mesuré à 25°C à 5% dans une solution aqueuse entre 7.8 et 9.2 et en ce que la composition comprend entre 0,2 g et 0,5 g de phase aqueuse/g de tensio-actif.

Procédé de fabrication de la base émulsionnante et composition cosmétique ou pharmaceutique la contenant.

## Description

### Domaine technique

L'invention concerne l'utilisation comme base émulsionnante dans une formulation cosmétique ou pharmaceutique, d'une composition comprenant, en tant que tensio-actif, du stéaroyl glutamate c'est-à-dire un mélange de stéaroyl glutamate de métal alcalin et d'acide stéaroyl glutamique, une phase grasse et une phase aqueuse. L'invention se rapporte également à une formulation cosmétique ou pharmaceutique intégrant une telle base émulsionnante de même qu'à un procédé de fabrication de ladite base émulsionnante.

### Art antérieur

Un certain nombre de bases émulsionnantes ont été développées pour le marché cosmétique ou pharmaceutique. Elles ont pour objectif d'entrer dans les formulations cosmétiques ou pharmaceutiques sous forme d'émulsion.

Le document du Demandeur WO2007/042723 décrit une base émulsionnante comprenant un dérivé de cires obtenu par réaction de cire de jojoba, de cire de riz, de cire de candelilla et de polyglycérol. La base comprend en outre de l'alcool cétostéarylique, du stéarate de glycérol et, en tant que tensio-actif, du stéaroyl lactylate de sodium.

Même si cette base confère des propriétés émulsionnantes satisfaisantes aux compositions qui la contiennent, la stabilité du tensio-actif au stockage avant préparation de la base, et notamment le risque d'hydrolyse du groupement lactylate, reste problématique. Par ailleurs, la présence d'une mauvaise qualité du dérivé lactylate peut conférer à l'émulsionnant une odeur indésirable.

Le document US2008/249192A1 décrit une base émulsionnante comprenant une cire anhydre contenant (a) un composé choisi parmi les esters de pentaerythritol, dipentaerythritol, et tripentaerythritol et (b) au moins un ester de glycérol et d'acides gras en C₁₂₋₂₄, le ratio en masse entre (a) et (b) étant de 1:3 à 3:1. La base contient en outre, en tant que tensio-actif, du stéaroyl glutamate monosodique ou disodique.

Le produit commercial vendu sous le nom de sodium stearoyl glutamate (SSG) disponible dans le commerce est un tensio-actif ionique composé d'une forme acide (acide stéaroyl glutamique) et d'une forme basique ionique (stéaroyl glutamate de sodium), dont le ratio varie en fonction de l'origine ou du fournisseur de la matière première.

La substitution du stéaroyl lactylate de sodium par le stéaroyl glutamate permet de résoudre les problèmes d'odeur et d'hydrolyse mais présente cependant certains inconvénients.

Le premier d'entre eux est que le stéaroyl glutamate de sodium est non soluble dans les phases grasses, alors que l'acide stéaroyl glutamique l'est, ce qui nécessite une agitation constante pour éviter que les particules sédimentent et est indispensable pour obtenir un mélange homogène.

Ce problème d'homogénéité rend par ailleurs infaisable l'obtention de formes solides divisées, par exemple pastilles ou écailles, qui soient elles-mêmes de composition homogène. En pratique, pour fabriquer des pastilles, il faut utiliser une pastilleuse qui doit être alimentée par un liquide homogène exempt de particules en suspension. De plus, la présence de ces particules peut contribuer à obturer les orifices et rendre le pastillage impossible. De plus le passage du réacteur à la pastilleuse se fait par des zones de transferts non agitées, dans lesquelles le SSG a tendance à sédimenter.

Le Demandeur a par ailleurs noté que les performances émulsifiantes du stéaroyl glutamate variaient d'une matière première à une autre, rendant difficile la reproductibilité du pouvoir émulsifiant des bases les incorporant d'un lot à l'autre ou d'un fournisseur à l'autre.

Plus précisément, le Demandeur a constaté que le stéaroyl glutamate, en fonction de son origine, ne présentait pas les mêmes performances tensioactives conférant aux formulations des viscosités et des stabilités différentes.

Le document WO 2019/042999 décrit des compositions de sprays solaires comprenant chacune un mélange de quatre ou cinq phases, une des phases comprenant du stéaroyl glutamate de sodium. Le pH des formulations cosmétiques finales est ajusté à l'aide d'un tampon tris, une fois les phases mélangées.

Le document US 2020/306151 décrit la capacité d'une ultra microémulsion à être épaissie en présence d'un dérivé de cellulose et de tensio-actifs non ioniques pour former une composition cosmétique destinée à être appliquée sur la peau. L'ultra microémulsion contient du stéaroyl glutamate de sodium. La composition contient de la soude qui sert à l'ajustement de son pH, une fois les différentes phases mélangées.

Le document FR3080766A1 décrit une composition cosmétique capillaire comprenant un dérivé de cires obtenu par la réaction de transestérification simultanée d'un mélange comprenant de la cire de jojoba et de la cire de tournesol, en présence du polyglycérol-3. Les exemples 12 et 13 décrivent respectivement une crème de nuit hydratante pour les cheveux et un masque capillaire fortifiant comprenant outre le dérivé de cire, du stéaroyl glutamate. La combinaison de la cire et du stéaroyl glutamate n'est pas décrite comme base émulsionnante.

Par conséquent, le problème que se propose de résoudre l'invention est celui de mettre au point une base émulsionnante à base de stéaroyl glutamate qui ne présente pas les inconvénients ci-avant.

En d'autres termes, le problème que se propose de résoudre l'invention est celui de mettre au point une base émulsionnante à base de stéaroyl glutamate, d'une phase grasse et d'une phase aqueuse :
- dont la forme liquide tout comme la forme solide soit homogène,
- qui présente des performances émulsifiantes équivalentes, quelle que soit l'origine du stéaroyl glutamate, c'est-à-dire qui permet d'obtenir une émulsion limpide à partir de la base émulsionnante, sans nécessité de réajuster le pH de ladite émulsion à l'exception des cas dans lesquels l'émulsion finale contient des ingrédients susceptibles de modifier le pH tels que par exemple, des acides.

### Exposé de l'invention

Le Demandeur a constaté que la combinaison d'une succession d'étapes d'agitation et de repos et d'une proportion de phase aqueuse précise permettant l'ajustement du taux de neutralisation du stéaroyl glutamate, permettait de parvenir, après mélange de l'ensemble des constituants, à une dissolution parfaite du stéaroyl glutamate de sodium dans la phase grasse.

Le Demandeur a également constaté qu'on obtenait des performances émulsifiantes constantes, quelle que soit l'origine du stéaroyl glutamate, à partir du moment où le pH du stéaroyl glutamate en solution à 5% en masse dans l'eau est compris entre 7.8 et 9.2.

Le Demandeur fait ainsi l'hypothèse que le ratio forme acide (stéaroyl glutamique) / forme basique (stéaroyl glutamate de sodium) contenu dans le stéaroyl glutamate avait une incidence sur les performances tensioactives du stéaroyl glutamate lorsqu'il était incorporé au sein d'une émulsion.

En d'autres termes, l'invention a pour objet l'utilisation comme base émulsionnante dans une formulation cosmétique ou pharmaceutique, d'une composition comprenant une phase grasse, une phase aqueuse, et un tensio-actif constitué d'un mélange de stéaroyl glutamate de métal alcalin et d'acide stéaroyl glutamique.

L'invention se caractérise en ce que le stéaroyl glutamate présente un pH mesuré à 25°C à 5% en masse dans une solution aqueuse entre 7.8 et 9.2.

Autrement dit, le Demandeur a constaté qu'il était nécessaire de mesurer et le cas échéant d'ajuster le ratio forme acide/forme basique du stéaroyl glutamate de sorte à obtenir un pH compris entre 7.8 et 9.2 dans les conditions ci-dessus pour disposer d'un stéaroyl glutamate dont les performances émulsionnantes soient robustes et reproductibles.

Le Demandeur a également constaté que le fait de mesurer le pH du stéaroyl glutamate seul et non celui de la base émulsionnante présentait l'avantage inattendu de pouvoir ajuster le pH de ladite base de manière beaucoup plus précise. Selon une première caractéristique, le métal alcalin du stéaroyl glutamate est du sodium ou du potassium.

Dans un mode de réalisation préféré, le tensio-actif contient du stéaroyl glutamate de sodium.

Selon une autre caractéristique, le tensio-actif représente entre 5 et 12%, avantageusement entre 7 et 9% en masse de la base émulsionnante, concentrations de tensio-actif auxquelles la base émulsionnante remplit les effets les meilleurs en termes de viscosité et de stabilité notamment.

Ces concentrations de tensio-actif permettent d'obtenir une émulsion limpide à partir de la base émulsionnante, sans nécessité de réajuster le pH hormis les cas, comme déjà mentionné, où certains ingrédients viennent perturber le pH de l'émulsion, du fait de leur caractère acide ou basique.

Pour assurer une solubilisation satisfaisante du stéaroyl glutamate, la phase aqueuse représente entre 1 et 5%, avantageusement entre 2 et 3,5% en masse de la base émulsionnante. En deçà de ces valeurs, le Demandeur a constaté que, soit il n'y a pas assez de phase aqueuse pour permettre la solubilisation complète du stéaroyl glutamate dans le corps gras, soit la totalité du stéaroyl glutamate est certes solubilisée mais un excès de phase aqueuse apparaît et décante au fond du mélangeur.

En pratique, la composition comprend entre 0,2 g et 0,5 g de phase aqueuse/g de tensio-actif.

Selon l'invention, a phase grasse contient des corps gras choisis dans la liste comprenant tels

Dans un mode de réalisation particulier, la phase grasse comprend un ou plusieurs corps gras solides à température ambiante, en pratique, dont le point de fusion est supérieur à 50°C.

De préférence, les corps gras, dont le point de fusion est supérieur à 50°C sont choisis dans le groupe comprenant les alcools gras, les glycérides partiels et/ou les dérivés de cires végétales modifiés par le polyglycérol.

Dans un mode de réalisation préféré, la phase grasse comprend un dérivé de cires obtenu par la réaction de transestérification simultanée d'un mélange comprenant de la cire de jojoba et de la cire de tournesol, en présence de polyglycérol-3.

En pratique, le dérivé de cire représente entre 20 et 40%, de préférence entre 24 et 35% en masse de la phase grasse.

Avantageusement, la phase grasse comprend en outre au moins un alcool gras en C₁₆-C₁₈ et du palmitostéarate de glycérol.

En pratique, le au moins un alcool gras en C₁₆-C₁₈ et le palmitostéarate de glycérol représentent respectivement entre 35 et 50% en masse de la phase grasse et entre 20 et 30% en masse de la phase grasse.

Dans un mode de réalisation particulier, attendu que la base émulsionnante de l'invention est homogène sous forme liquide à chaud (c'est à dire lorsque le point de fusion de la phase grasse est supérieur à 50°C), celle-ci peut être facilement pastillée pour se présenter sous forme de particules solides individualisées et notamment sous la forme d'écailles ou de pastilles de taille supérieure ou égale à 1 millimètre.

L'invention a également pour objet une formulation cosmétique ou pharmaceutique, en particulier topique, comprenant la base émulsionnante telle que décrite précédemment.

En pratique, la base émulsionnante représente entre 1 et 8%, de préférence entre 2 et 5% en masse de la composition.

La base émulsionnante de l'invention peut être mise en oeuvre dans toutes les formes galéniques normalement utilisées pour une application topique sur la peau, les lèvres ou les cheveux, notamment au sein d'une composition cosmétique sous forme d'une émulsion huile-dans-eau ou eau-dans-huile ou multiple, d'une émulsion siliconée, d'une microémulsion ou nano-émulsion.

La composition peut être plus ou moins fluide et avoir l'aspect entre autres d'une crème blanche ou colorée, d'une pommade, d'un lait, d'une lotion, d'un sérum. La composition peut également être conditionnée dans un aérosol et peut être sous forme de mousse, de spray ou de stick.

La composition peut contenir les adjuvants habituels dans les domaines cosmétique et dermatologique, tels que les matières grasses, les émulsionnants et co-émulsionnants, les gélifiants hydrophiles ou lipophiles, les actifs hydrophiles ou lipophiles, les conservateurs, les antioxydants, les solvants, les parfums, les charges, les filtres solaires hydrophiles et lipophiles, les matières colorantes, les bases et acides, les agents propénétrants, et les polymères.

Les quantités de ces différents adjuvants sont celles classiquement utilisées dans les domaines considérés, et par exemple de 0.01 à 40% de la masse totale de la composition. Ces adjuvants, selon leur nature, peuvent être introduits dans la phase grasse ou dans la phase aqueuse.

Comme matières grasses utilisables dans la composition cosmétique de l'invention, on peut utiliser les huiles minérales, les huiles d'origine animale (lanoline), les huiles végétales, les huiles de synthèse (isopropyl myristate, octyldodecanol, isostearyl isostearate, decyl oleate, isopropyl palmitate, caprylic/capric triglycérides), les huiles siliconées (cyclomethicone, dimethicone). On peut utiliser comme matières grasses des alcools gras, des acides gras, des cires, des beurres, des huiles hydrogénées et des gommes et en particulier les élastomères de silicone.

Comme émulsionnants et co-émulsionnants utilisables dans la composition cosmétique de l'invention, on peut citer des tensio-actifs non ioniques, ioniques (anioniques ou cationiques) ou zwitterioniques, comme par exemple les esters de polyglycérols et d'acides gras, les esters de saccharose et d'acides gras, les esters de sorbitane et d'acides gras, les esters d'acides gras et de sorbitan polyoxyéthylénés, les éthers d'alcools gras et de PEG, les esters de glycérol et d'acides gras, les alkyl sulfates, les alkyl éther sulfates, les alkyl phosphates, les alkyl polyglucosides, les alkyl polypentosides, les dimethicones copolyols, et les dérivés de l'acide citrique.

Comme gélifiants hydrophiles, on peut citer en particulier les polymères carboxyvinyliques, les copolymères acryliques(carbomer) tels que les copolymères d'acrylates/alkylacrylates, les copolymères d'acide 2-acrylamido-2-méthylpropane, les polyacrylamides, les polysaccharides tels que la gomme xanthane, la gomme guar, les carraghénanes, les gommes naturelles tels que la gomme de cellulose et ses dérivés, les amidons et leurs dérivés, les argiles.

Comme gélifiants lipophiles, on peut citer les argiles modifiées comme les bentones, les sels métalliques d'acides gras, la silice et ses dérivés, l'éthylcellulose, les polyamides, les esters de dextrose.

La composition cosmétique peut également contenir des actifs. Comme actifs, on peut utiliser notamment les dépigmentants, , les agents hydratants, les antioxydants, les anti-séborrhéiques, les anti-inflammatoires, les anti-acnéiques, les agents kératolytiques et/ou desquamants, les actifs anti-rides et tenseurs, les actifs drainants, les agents anti-irritants, les agents apaisants, les amincissants tels que les bases xanthiques (caféine), les vitamines et leurs mélanges, les agents matifiants, les actifs anti-âge tel que le rétinol, et les huiles essentielles.

Comme conservateurs utilisables selon l'invention, on peut citer l'acide benzoïque, ses sels et ses esters ; l'acide sorbique et ses sels ; les parabens, leurs sels et esters ; le triclosan ; l'imidazolidinyl urée ; le phénoxyéthanol ; la DMDM hydantoïne ; le diazolidinyl urée ; la chlorphenesin ; l'alcool benzylique ; l'acide dehydroacétique ; l'acide levulinique et dérivés ; l'acide salicylique et ses sels ; l'ethylhexylglycerin ; des acides organiques tels que le gluconolactone.

Comme antioxydants utilisables selon l'invention, on peut citer les agents chélatants tels que l'EDTA et ses sels.

Comme solvants utilisables selon l'invention, on peut citer l'eau, l'éthanol, la glycérine, le propylène glycol, le butylène glycol, le propanediol, le pentylène glycol, l'hexanediol.

Comme charges utilisables selon l'invention, on peut citer le talc, le kaolin, le mica, la sérécite, le magnésium carbonate, l'aluminium silicate, le magnésium silicate, les poudres organiques telles que le nylon, le PMMA, les amidons, des poudres naturelles telles que la poudre de riz. Ces charges peuvent également être modifiées chimiquement ou enrobées pour modifier leurs caractéristiques.

Comme filtres solaires utilisables selon l'invention, on peut citer les filtres UVA et UVB classiquement utilisés tels que les benzophenones, le butyl methoxydibenzoyl methane, l'octocrylène, l'octyl methoxycinnamate, le 4-methylbenzylidene camphor, l'octyl salicylate, le tacephthalydene dicamphor sulfanic acid, le phenylbenzimidazolesulfonic acid, l'homosalate, le 2-(4-diethylamino-2-hydroxybenzoyl)benzoate, le disodium phenyldibenzimidazoletetrasulfonate, le Methylenebis-benzotriazolyl tetramethylbutylphenol, les dérivés triazine, le Polysilicone-15 et le drométrizole trisiloxane. On citera également les filtres physiques TiO2 et ZnO sous leurs formes micrométriques et nanométriques, enrobés ou non enrobés.

Comme matières colorantes utilisables selon l'invention, on peut citer les colorants lipophiles, les colorants hydrophiles, les pigments et les nacres habituellement utilisés dans les compositions cosmétiques ou dermatologiques, et leurs mélanges.

Comme bases et acides utilisables selon l'invention, on peut citer la soude, la triéthanolamine, l'aminométhyl propanol, l'hydroxyde de potassium, l'acide citrique, l'acide lactique, l'acide phosphorique.

Comme agents propénétrants utilisables selon l'invention, on peut citer les alcools et glycols (éthanol, propylène glycol), l'éthoxydiglycol, les alcools et acides gras (acide oléique), les esters d'acides gras, le diméthyl isosorbide.

La composition selon l'invention peut être utilisée comme produit de soin, comme produit de nettoyage, et/ou comme produit de maquillage de la peau, comme produit de protection solaire, ou comme produit capillaire.

L'invention concerne également un procédé de fabrication de la base émulsionnante ci avant décrite, selon lequel :
- on prépare la phase grasse à l'état liquide,
- on introduit le tensio-actif dans la phase grasse, en pratique la totalité du tensio-actif, sous agitation,
- avant ou après ajout du tensio-actif et avant d'ajouter l'eau dans la phase grasse, on mesure séparément le pH à 5% dans l'eau, à 25°C, du stéaroyl glutamate, c'est-à-dire indépendamment de la phase grasse,
- le cas échéant, on ajuste le ratio acide stéaroyl glutamique/ stéaroyl glutamate de sodium en ajustant le pH du tensio-actif à 5%, à 25°C, dans l'eau par ajout d'une base ou acide pour obtenir un pH compris entre 7.8 et 9.2.
- lorsque l'ajout d'une base ou d'un acide est nécessaire, on détermine la masse de base ou acide à ajouter en fonction de la quantité de tensio-actif mise en oeuvre lors la fabrication de la base émulsionnante, c'est-à-dire la quantité de tensio-actif introduite dans la phase grasse,
- on prépare la phase aqueuse en ajoutant la masse de base ou d'acide déterminée dans l'étape précédente,
- on ajoute à la phase grasse entre 0,2 g et 0,5 g de phase aqueuse/g de tensio-actif,
- on alterne des phases d'agitation et de repos jusqu'à dissolution complète du tensio-actif.

Comme déjà souligné, l'ajustement du pH du tensio-actif est plus précis en déterminant la masse de base ou d'acide nécessaire, pour ensuite ajouter ladite masse de base ou d'acide dans la phase grasse plutôt que d'ajuster le pH de la base émulsionnante une fois fabriquée.

Dans le mode de réalisation selon lequel le point de fusion des composants de la phase grasse est supérieur, en pratique à 50°C, l'étape de préparation de la phase grasse à l'état liquide est effectuée par chauffage desdits composants à une température supérieure à leur point de fusion.

Selon un mode de réalisation préféré, les phases d'agitation durent entre 5 et 15 min, de préférence 10 min alors que les phases de repos durent entre 2 et 10 min, de préférence, 5 min.

Avantageusement, on répète au moins 2 fois, de préférence on répète 4 fois les phases d'agitation et de repos.

Dans un mode de réalisation particulier, selon lequel le point de fusion des composants de la phase grasse est supérieur, en pratique à 50°C, la base émulsionnante est soumise à une étape de pastillage.

L'invention et les avantages qui en découlent ressortiront bien des exemples de réalisation suivants à l'appui des figures annexées.

### Brève description des dessins

La figure 1 (1a à 1g) est une série de photographies des produits obtenus à l'issue des différentes étapes du procédé de fabrication de la base émulsionnante.

La figure 2 est une photographie de la base émulsionnante de l'invention sous forme de pastilles.

### Description détaillée de l'invention

Composition de la base émulsionnante

La composition de la base émulsionnante est reproduite dans le tableau 1 ci-dessous.

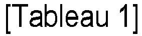

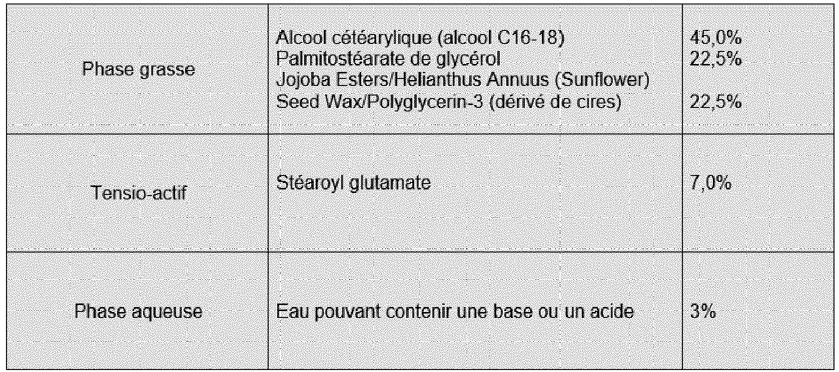

### Procédé de fabrication de la base émulsionnante

La base émulsionnante est préparée de la façon suivante.

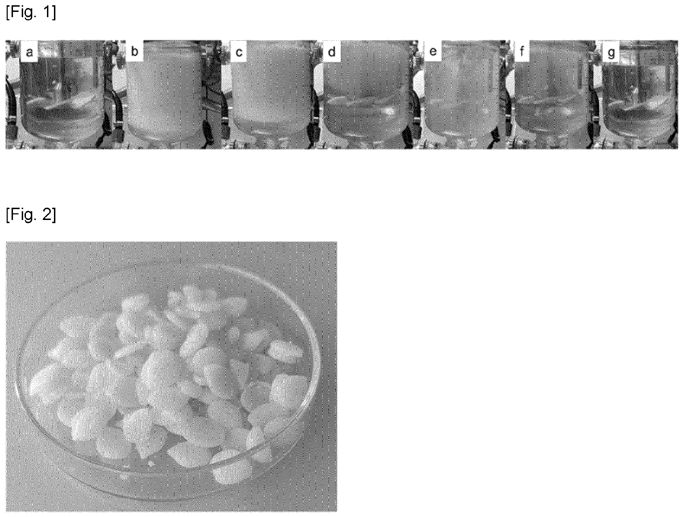

On mélange les différents composés de la phase grasse puis on fait fondre le mélange à 80°C. On agite ensuite la phase liquide obtenue à raison de 250 trs/min pour un réacteur d'un litre. La figure 1a est une photographie de la phase obtenue à ce stade du procédé.

Le stéaroyl glutamate est ensuite lentement introduit dans la phase grasse, toujours sous agitation. L'agitation du mélange permet une bonne dispersion de la poudre. La dispersion est représentée sur la Figure 1b.

On mesure séparément le pH à 5%, à 25°C, dans l'eau du stéaroyl glutamate. Le cas échéant, on ajuste le ratio acide stéaroyl glutamique/ stéaroyl glutamate de sodium en ajustant le pH du tensio-actif à 5%, à 25°C, dans l'eau par ajout d'une base ou acide pour obtenir un pH compris entre 7.8 et 9.2. Dans ce cas, la masse de base ou acide devant être ajoutée est extrapolée à la quantité de tensio-actif mise en oeuvre lors la fabrication de la base émulsionnante. La phase aqueuse est préparée par l'ajout de la masse de base ou acide déterminée ci-avant.
On ajoute ensuite la phase aqueuse à la dispersion de la figure 1b. La quantité de phase aqueuse ajoutée est comprise entre 0,2 g et 0,5 g d'eau/g de stéaroyl glutamate. En dessous de 0,2 g d'eau/g de stéaroyl glutamate, il n'y a pas assez d'eau pour permettre la solubilisation complète du stéaroyl glutamate dans la phase grasse. Au-dessus de 0,5 g d'eau/g de stéaroyl glutamate, la totalité du stéaroyl glutamate est solubilisée, mais un excès d'eau apparaît et décante au fond du mélangeur.

La solubilisation du stéaroyl glutamate dans la phase grasse se fait en plusieurs étapes d'agitation et arrêt de l'agitation (repos).

Une fois l'eau ajoutée, la dispersion, dans cet exemple, est agitée pendant 10 minutes puis mise au repos pendant 5 minutes (Figure 1c).

Cette étape d'agitation de 10 minutes et repos de 5 minutes est répétée entre 1 et 4 fois (Figures 1 d, e, f) jusqu'à l'obtention d'un produit limpide homogène (Figure 1g).

Ce mélange homogène et limpide peut être gardé à 90°C pendant 48h dans un contenant clos sans changement d'aspect macroscopique ou de composition chimique.

### Pastillage

Une fois la base émulsionnante limpide, celle-ci peut être pastillée sans difficulté pour obtenir des pastilles homogènes entre elles sur toute la durée de l'opération de pastillage mais aussi au sein d'une même pastille (Figure 2).

### Essais comparatifs

On prépare 3 formulations qui diffèrent les unes des autres uniquement par le pH du tensio-actif mesuré à 5% dans l'eau. La base émulsionnante est celle du tableau 1, après ajustement ou non du pH du tensio-actif à 5% dans l'eau.

La composition des formulations est représentée dans le tableau 2. Les proportions des ingrédients sont données en pourcentage en masse.

**[Tableau 2]**

| | **Ingrédients** | **Formulation 1** | **Formulation 2** | **Formulation 3** |
|---|---|---|---|---|
| Phase grasse | Polydécène hydrogéné | 4.5% | 4.5% | 4.5% |
| | Triglycérides en C8/C10 | 4.5% | 4.5% | 4.5% |
| | Isostéarate d'isostéaryle | 3% | 3% | 3% |
| | Huile de noyaux d'abricot | 3% | 3% | 3% |
| | Phenoxyethanol | 0.72% | 0.72% | 0.72% |
| | Ethylhexylglycerin | 0.08% | 0.08% | 0.08% |
| Emulsionnant | Emulsionnant selon tableau 1 conforme à l'invention (pH à 5% dans l'eau du tensio-actif entre 7.8 et 9.2) | 6% | | |
| | Emulsionnant selon tableau 1 non conforme à l'invention (pH à 5% dans l'eau du tensio-actif < 7.8) | | 6% | |
| | Emulsionnant selon tableau 1 non conforme à l'invention (pH à 5% du tensio-actif > 9.2) | | | 6% |
| Phase aqueuse | Eau | 78.2% | 78.2% | 78.2% |
| 24h | Aspect | Crème blanche brillante | Crème blanche brillante | Crème blanche brillante |
| | Viscosité (cP) | 52 000 | 40 800 | 25 600 |
| | pH | 7,3 | 6,3 | 7,8 |
| 1 semaine | Aspect | conforme | conforme | conforme |
| | Viscosité (cP) | 56 300 | 38 600 | 30 600 |
| | pH | 7,4 | 6,3 | 7,4 |
| | Aspect 40° | conforme | conforme | conforme |
| 1 mois | Aspect | conforme | conforme | conforme |
| | Viscosité (cP) | 51 500 | 39 600 | 22 800 |
| | pH | 7,6 | 6,3 | 7,1 |
| | Aspect à 40°C | conforme | non conforme Début de crémage (2s) | conforme |
| 3 mois | Aspect | conforme | conforme | conforme |
| | Viscosité (cP) | 47 300 | 31 400 | 14 400 non conforme |
| | pH | 7,5 | 6,3 | 6,8 non conforme |
| | Aspect à 40°C | conforme | non conforme | conforme |

Il ressort de ce qui précède que la formule 1, dont le pH à 5% dans l'eau du tensio-actif est compris entre 7.8 et 9.2, présente une viscosité proche de 50 000 cP, ce qui correspond à ce qui est attendu. Les résultats montrent également que la stabilité de la formule 1 est bonne au cours du temps.

### Exemples de formulations cosmétiques

### Sérum anti-âge

**[Tableau 3]**

| ***Ingrédient*** | ***% m*/*m*** |
|---|---|
| Eau déminéralisée | Qsp 100.0 |
| Hydroxypropyl guar | 1.0 |
| Glycérine | 2.0 |
| Gomme xanthane | 0.2 |
| Pullulan | 2.0 |
| BASE EMULSIONNANTE SELON L'INVENTION | 3.0 |
| Conservateur | 0.7 |
| Undecane, tridecane | 10.0 |
| Actif anti-âge | 1.0 |
| Nacres | 0.5 |
| Acide citrique | qs |

### Lait corps solaire FPS 30

**[Tableau 4]**

| ***Ingrédient*** | ***% m*/*m*** |
|---|---|
| Eau déminéralisée | Qsp 100.00 |
| Sphingomonas Ferment Extract | 0,15 |
| Ethylhexyl Triazone | 3,00 |
| Diethylamino Hydroxybenzoyl Hexyl Benzoate | 5,00 |
| Bis-Ethylhexyloxyphenol Methoxyphenyl Triazine | 2,00 |
| Ethylhexyl Salicylate | 5,00 |
| Diisopropyl Adipate, Ethylcellulose | 1,50 |
| Dibutyl Adipate | 6,00 |
| Diisopropyl Sebacate | 9,00 |
| Butyl Octyl Salicilate | 2,50 |
| Phenoxyethanol, Ethylhexylglycerin | 0,70 |
| Béhénate de glycérol | 1,50 |
| BASE EMULSIONNANTE SELON L'INVENTION | 5,00 |
| Diisostearoyl polyglyceryl-3 dimer dilinoleate | 2,00 |
| Copemicia cerifera (camauba) wax, oryza sativa (rice) bran wax | 3,00 |
| Butylène glycol | 4,00 |
| Actif hydratant | 2,00 |
| Acide citrique | qs |

### Crème de jour

**[Tableau 5]**

| ***Ingrédient*** | ***% m*/*m*** |
|---|---|
| Alcool cétylique | 3.0 |
| Jojoba esters, helianthus annuus (sunflower) seed wax, acacia decurrens flower wax, potyglycerin-3 | 2.0 |
| C10-18 triglycerides | 5.0 |
| Dicaprylyl ether | 18.0 |
| Huile végétale hydrogénée | 5.0 |
| Conservateur | 1.0 |
| BASE EMULSIONNANTE SELON L'INVENTION | 5.0 |
| Eau déminéralisée | Qsp 100.0 |
| Acacia senegal gum, xanthan gum | 0.4 |
| Glycérine | 5.0 |
| Acide citrique | 0.5 |
| Actif tenseur | 1.0 |
| Parfum | 0.2 |
| Nacres | 1.2 |
| Agent matifiant | 2.0 |

## Revendications

1. Utilisation comme base émulsionnante dans une formulation cosmétique ou pharmaceutique, d'une composition comprenant une phase grasse, une phase aqueuse, et un tensio-actif contenant un mélange de stéaroyl glutamate de métal alcalin et d'acide stéaroyl glutamique, **caractérisée en ce que** le tensio-actif présente un pH mesuré à 25°C dans une solution à 5% en masse dans l'eau compris entre 7.8 et 9.2 et **en ce que** la composition comprend entre 0,2 g et 0,5 g de phase aqueuse/g de tensio-actif.

2. Utilisation selon la revendication 1, **caractérisée en ce que** le métal alcalin est le sodium.

3. Utilisation selon la revendication 1, **caractérisée en ce que** le stéaroyl glutamate de métal alcalin est le stéaroyl glutamate de sodium.

4. Utilisation selon l'une des revendications précédentes, **caractérisée en ce que** le tensio-actif représente entre 5 et 12%, avantageusement entre 7 et 9% en masse de la base émulsionnante.

5. Utilisation selon l'une des revendications précédentes, **caractérisée en ce que** la phase grasse comprend un dérivé de cires obtenu par la réaction de transestérification simultanée d'un mélange comprenant de la cire de jojoba et de la cire de tournesol, en présence de polyglycérol-3.

6. Utilisation selon la revendication 5, **caractérisée en ce que** le dérivé de cires représente entre 20 et 40%, de préférence entre 24 et 35% en masse de la phase grasse.

7. Utilisation selon l'une des revendications 5 ou 6, **caractérisée en ce que** la phase grasse comprend en outre au moins un alcool gras en C₁₆-C₁₈ et du palmitostéarate de glycérol.

8. Utilisation selon la revendication 7, **caractérisée en ce que** le au moins un alcool gras en C₁₆-C₁₈ et le palmitostéarate de glycérol représentent respectivement entre 35 et 50% en masse de la phase grasse et 20 à 30% en masse de la phase grasse.

9. Utilisation selon l'une des revendications précédentes, **caractérisée en ce que** la composition se présente sous forme de particules solides individualisées.

10. Utilisation selon la revendication 9, **caractérisée en ce que** les particules solides individualisées se présentent sous la forme d'écailles ou de pastilles de taille supérieure ou égale à 1 millimètre.

11. Composition cosmétique comprenant la base émulsionnante telle que décrite dans l'une des revendications 1 à 10.

12. Composition cosmétique selon la revendication 11, **caractérisée en ce que** la base émulsionnante représente entre 1 et 8% en masse de la composition, de préférence entre 2 et 5% en masse.

13. Procédé de fabrication d'une base émulsionnante pour formulation cosmétique ou pharmaceutique comprenant une phase grasse, une phase aqueuse, et en tant que tensio-actif, un mélange de stéaroyl glutamate de sodium et d'acide stéaroyl glutamique, selon lequel :
- on prépare la phase grasse à l'état liquide,
- on introduit le tensio-actif dans la phase grasse, sous agitation,
- avant ou après ajout du tensio-actif et avant d'ajouter l'eau dans la phase grasse, on mesure séparément le pH à 5% dans l'eau, à 25° C, du stéaroyl glutamate,
- le cas échéant, on ajuste le ratio acide stéaroyl glutamique/ stéaroyl glutamate de sodium en ajustant le pH du tensio-actif à 5% dans l'eau, à 25°C, par ajout d'une base ou acide pour obtenir un pH compris entre 7.8 et 9.2,
- lorsque l'ajout d'une base ou d'un acide est nécessaire, on détermine la masse de base ou acide à ajouter en fonction de la quantité de tensio-actif mise en oeuvre lors la fabrication de la base émulsionnante,
- on prépare la phase aqueuse en ajoutant la masse de base ou d'acide déterminée dans l'étape précédente,
- on ajoute à la phase grasse entre 0,2 g et 0,5 g de phase aqueuse/g de tensio-actif,
- on alterne des phases d'agitation et de repos jusqu'à dissolution complète du tensio-actif.

14. Procédé selon la revendication 13, **caractérisé en ce que** la base émulsionnante est soumise à une étape de pastillage.
